# FASCICULE DE BREVET EUROPEEN

(11) **EP 0 899 267 B1**
(45) Date de publication et mention de la délivrance du brevet: **11.12.2002**
(21) Numéro de dépôt: 98402155.0
(22) Date de dépôt: 01.09.1998
(51) Int. Cl.: C07D 265/34, A61K 31/535

(54) **Nouvelles trans-3,4,4a,5,6,10b-hexahydro-2H-napht(1,2-b)-1,4-oxazines disubstituées, leur procédé de préparation et les compositions pharmaceutiques qui les contiennent**
Disubstituierte trans-3,4,4a,5,6,10b-Hexahydro-2H-napht(1,2-b)-1,4-Oxazine, Verfahren zu deren Herstellung und diese enthaltende pharmazeutigsche Zusammensetzungen
Disubstituted trans-3,4,4a,5,6,10b-hexahydro-2H-napht(1,2-b)-1,4-oxazines, process for their preparation and pharmaceutical compositions containing them

(30) Priorité: 01.09.1997 FR 9710852
(43) Date de publication de la demande: 03.03.1999
(73) Titulaire: LES LABORATOIRES SERVIER, 92200 Neuilly sur Seine (FR)
(72) Inventeur: Peglion, Jean-Louis, 78110 Le Vesinet (FR); Harmange, Jean-Christophe, Andover MA 01810 (US); Millan, Mark, 78230 Le Pecq (FR); Lejeune, Françoise, 92210 Saint Cloud (FR)

(56) Documents cités:
- EP-A- 0 080 115
- WO-A-91/19719
- US-A- 4 540 691
- US-A- 5 486 611

## Description

La présente invention a pour objet de nouvelles trans-3,4,4a,5,6,10b-hexahydro-2H-napht[1,2-b]-1,4-oxazines disubstituées, leur procédé de préparation et les compositions pharmaceutiques qui les contiennent.
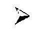 Elle concerne plus spécialement les composés de formule I : 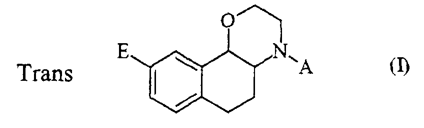 dans laquelle :
   ◆ A représente :
      un radical (C₁-C₁₀)alkyle, (C₃-C₁₀)alcényle ou (C₃-C₁₀)alcynyle, chacun en chaîne droite ou ramifiée et chacun éventuellement substitué par un ou plusieurs radicaux cycloalkyles ayant de 3 à 8 atomes de carbone, ou par un radical aryle choisi parmi les radicaux phényle, thiényle et pyridyle chacun d'eux éventuellement substitué par un ou plusieurs substituants choisis parmi les atomes d'halogène, le radical hydroxy et les radicaux alkyle et alkoxy ayant chacun de 1 à 6 atomes de carbone en chaîne droite ou ramifiée.
   ◆ E représente : 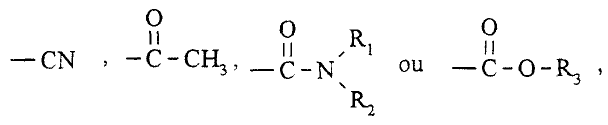 dans lesquels :
      - R₁ et R₂, identiques ou différents, représentent chacun un atome d'hydrogène ou prennent la signification de A précédemment définie, et
      - R₃ représente un atome d'hydrogène ou un radical alkyle de 1 à 5 atomes de carbone en chaine linéaire ou ramifiée ;

      - sous forme racémique ou d'isomères optiques et leurs sels d'addition avec un acide pharmaceutiquement acceptable.
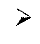 Les composés de formule I agissent comme de puissants ligands dopaminergiques tant in vitro qu'in vivo.

Les dérivés dopaminergiques sont largement utilisés en thérapeutique grâce à leurs effets bénéfiques dans les maladies psychiatriques et en périphérie dans les maladies cardiovasculaires.

Cinq sous-types réceptoriels dopaminergiques (D₁ à D₅) ont été actuellement clonés et caractérisés. Actuellement, la grande majorité des médicaments agissent au niveau du système dopaminergique par l'intermédiaire de leur action sur le sous-type D₂, qu'il s'agisse de bloqueurs (ou antagonistes) ou d'activateurs (ou agonistes). Ces médicaments présentent de nombreux effets secondaires : dyskinésie tardive, hyperprolactinémie, aménorrhée pour les premiers, effets cardio-vasculaires et moteurs pour les seconds.

Contrairement aux récepteurs D₂, la concentration des récepteurs D₃ est très faible dans le noyau nigrostrié et dans les cellules lactotrophes. Par contre, à l'instar des récepteurs D₂ la concentration des récepteurs D₃ est très importante au niveau du système limbique. Cette différence importante dans la localisation de ces deux sous-types réceptoriels encourage la recherche de nouveaux médicaments agissant de façon préférentielle sur le sous-type D₃ et qui devrait se traduire par une minimisation ou une disparition des effets secondaires liés typiquement au sous-type D₂, comme mentionné précédemment.

Très peu de produits revendiquent actuellement un tel mécanisme d'action.

L'état antérieur de la technique fait état toutefois de dérivés tricycliques de l'aminotétraline (cf le brevet US 5.486611), mais ces dérivés agissent préférentiellement au niveau du système sérotoninergique et plus précisément au niveau du sous-type réceptoriel 5HT_{1A}. Les dérivés de la présente invention se différencient de ces produits à la fois par leur structure mais aussi par leur spécificité d'action.

Les études réalisées in vitro (binding sur récepteurs humains clonés D₂ et D₃) avec les composés de la présente invention montrent que ceux-ci se comportent comme des ligands préférentiels des récepteurs D₃ avec une moindre affinité pour le récepteur D₂.

Cette caractéristique confère un intérêt tout particulier aux composés de la présente invention dû à leur faible niveau d'expression d'effets secondaires.

Plusieurs tests in vivo ont confirmé leur mécanisme d'action (enregistrement de l'activité électrique unitaire, extracellulaire dans l'aire tegmentale ventrale du rat) et l'intérêt de leur utilisation dans le traitement de nombreuses maladies du système nerveux central.

Les composés de l'invention ont démontré notamment leur activité dans le test des vocalisations ultrasoniques chez le rat, le test de la nage forcée et dans le test de rotation chez les rats lésés au 6-OH-DPAT.

Les résultats obtenus permettent donc de proposer les produits de l'invention dans le traitement de l'anxiété, de la dépression, de l'agressivité, de la maladie de Parkinson et de la schizophrénie.
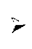 La présente invention a également pour objet le procédé de préparation des composés de formule I caractérisé en ce que :
   - l'on fait réagir un composé trans de formule II : 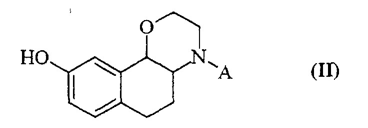 dans laquelle A a la signification précédemment définie,
   - avec de l'anhydride triflique en présence de pyridine pour obtenir un composé trans de formule III : 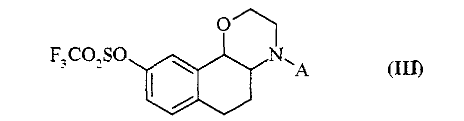 dans laquelle A a la signification précédemment définie,
   - et l'on fait réagir ce composé :
      . soit avec du cyanure de zinc et du tétrakis (triphénylphosphine)palladium (o) dans le diméthylformamide à chaud, pour obtenir un composé trans de formule la : 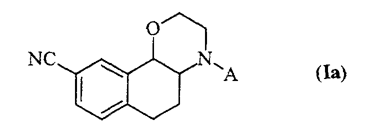
      dans laquelle A a la signification précédemment définie,
      . soit avec du n-butyl vinyl éther dans les conditions de la réaction de Heck pour obtenir un composé trans de formule Ib : 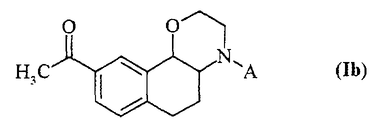 dans laquelle A a la signification précédemment définie,
      et, si on le désire,
   - le composé la, précédemment obtenu, est traité par un mélange d'acide chlorhydrique et d'acide acétique au reflux, pour obtenir un composé trans de formule Ic : 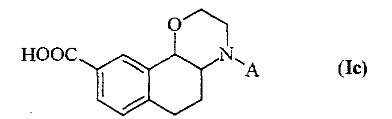 dans laquelle A a la signification précédemment définie,
   - lequel composé Ic peut être traité :
      . soit, par un composé de formule IV :

         R₃-OH (**IV**)

         dans laquelle R₃ a la signification précédemment définie,
         en présence d'acide chlorhydrique, pour obtenir un composé trans de formule Id : 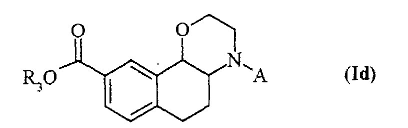 dans laquelle A et R₃ ont les significations précédement définies,
      . soit par un composé de formule V : 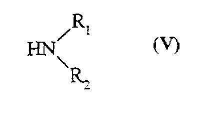 dans laquelle R₁ et R₂ ont les significations précédemment définies,
      en présence de 2-(1H-benzotriazol-1-yl) 1,1,3,3-tétraméthyluronium tétrafluoroborate, de triéthylamine et d'une quantité catalytique de 4-diméthylaminopyridine dans le chlorure de méthylène, pour obtenir un composé trans de formule Ie : 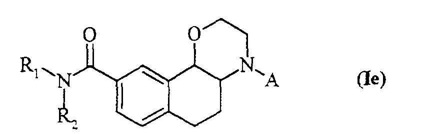 dans laquelle A, R₁, et R₂, ont les significations précédemment définies.

L'ensemble des composés trans de formule la, Ib, Ic, Id, et le forme l'ensemble des composés trans de formule I.

Les matières premières de formule II ont été préparées selon des procédés décrits dans la littérature, à partir de substances connues.

Les formes optiquement actives des composés de formule I ont été obtenues soit à partir de formes optiquement actives des matières premières de formule II, soit par dédoublement des formes racémiques dès composés de formule I, selon des méthodes connues de la littérature.

La présente invention a également pour objet les compositions pharmaceutiques contenant comme principe actif un composé de formule I ou un de ses sels physiologiquement tolérable, mélangé ou associé à un ou plusieurs excipients pharmaceutiques appropriés.

Les compositions pharmaceutiques ainsi obtenues se présentent généralement sous forme dosée renfermant de 0,5 à 25 mg de principe actif. Elles peuvent, par exemple, revêtir la forme de comprimés, dragées, gélules, suppositoires, solutions injectables ou buvables et être administrées par voie orale, rectale ou parentérale.

La posologie peut varier selon l'âge et le poids du patient, la voie d'administration, la nature de la maladie et les traitements associés et s'échelonne de 0,5 à 25 mg de principe actif, 1 à 3 fois par jour.

Les exemples suivants, donnés à titre non-limitatif, illustrent la présente invention. Les points de fusion ont été déterminés soit à la platine chauffante de Kofler (K), soit à la platine chauffante sous microscope (MK). Les spectres de résonnance magnétique nucléaire du proton (RMN) ont été réalisés à 200 MHz, sauf indication contraire.

### Exemple 1 : Trans-3,4,4a,5,6,10b-hexahydro-9-cyano-4-propyl-2H-naphte[1,2-b]-1,4-oxazine et son chlorhydrate

### Stade A : trans-3,4,4a,5,6,10b-hexahydro-9-[(trifluorométhyl)sulfonyloxy]-4-propyl-2H-naphto[1,2-b]-1,4-oxazine

18,7 ml d'anhydride triflique sont additionnés lentement à une solution de 21,0g de trans-3,4,4a,5,6,10b-hexahydro-9-hydroxy-4-propyl-2H-naphto[1,2-b]-1,4-oxazine dans 17 ml de pyridine en maintenant la température entre 0° C et 5° C. Après 1 heure d'agitation à 0° C, le mélange réactionnel est concentré sous vide. Le résidu d'évaporation est chromatographié sur colonne de silice (éluant : CH₂Cl₂/CH₃COOC₂H₅ ; 85/15) pour donner 22,4 g du produit attendu.
P.F. : 56°-57°C (K)
Rendement : 69 %

### Stade B : composé titre

2,39 g de Zn(CN)₂ et 1,34 g de Pd[P(C₆H₅)₃]₄ sont successivement additionnés à température ambiante à une solution dégazée de 11,0g du composé titre du stade A dans 55 ml de diméthylformamide (DMF). Le mélange réactionnel est chauffé 3 heures à 80° C. Puis 2,39 g de Zn(CN)₂ et 1,34 g de Pd[P(C₆H₅)₃]₄ y sont de nouveau successivement additionnés à 80° C. Après 18 heures de chauffage à 80° C, le mélange réactionnel est versé sur 500 ml d'eau. Le mélange est extrait à l'éther éthylique. Les phases organiques sont rassemblées, et extraites quatre fois par HCI 1N. Les phases aqueuses sont rassemblées, alcalinisées par de la lessive de soude et extraites à l'éther éthylique. Les phases organiques sont rassemblées, lavées une fois par de la saumure, séchées sur sulfate de magnésium et concentrées sous vide pour donner 6,27 g du produit désiré. 1 g de ce produit est chromatographié sur colonne de silice (éluant : CH₂Cl₂/CH₃COOC₂H₅ ; 90/10) pour donner 0,66 g de base attendue dont le chlorhydrate est cristallisé de l'acétate d'éthyle.
P.F. : 201-203° C (MK) (sublimation 190-195° C)

### R.M.N.: ¹H 300 MHz (DMSO)d₆

11,6 ppm, m, 1H; 7,75 ppm, d, 1H; 7,7 ppm, dd, 1H; 7,4 ppm, d, 1H; 5,0 ppm, d, 1H; 4,25 ppm, d, 2H; 3,6 ppm, d, 1H; 3,45-3,15 ppm, m, 3H; 3,0 ppm, m, 3H; 2,5 ppm, m, 1H; 1,75 ppm, m, 2H; 0,95 ppm, t, 3H.

### Exemple 2 : Trans-3,4,4a,5,6,10b-hexahydro-9-carbamoyl-4-propyl-2H-naphte[1,2-b]-1,4-oxazine

Une solution de 1 g du produit titre de l'exemple 1 dans 37 ml d'éthanol et 0,6 g de KOH est chauffée à reflux. Après 48h, le mélange réactionnel est concentré sous vide. Le résidu est repris par 200 ml d'un mélange CH₂Cl₂/CH₃OH: 95/5. La solution, lavée deux fois par de la saumure, séchée sur sulfate de magnésium est concentrée sous vide pour donner 0,8 g d'un solide qui recristallise du méthanol.
P.F. : 200-202°C (MK)
Rendement : 72%

### R.M.N.: ¹H (DMSO)d₆

7,94 ppm, s, 1H; 7,90 ppm, s, 1H (échangeable); 7,66 ppm, dd, 1H; 7,15 ppm, s, 1H (échangeable); 7,14 ppm, d, 1H; 4,20 ppm, d, 1H; 4,00 ppm, dd, 1H; 3,78 ppm, m, 1H; 2,98-2,70 ppm, m, 4H; 2,40-2,0 ppm, m, 4H; 1,60-1,35 ppm, m, 3H; 0,9 ppm, t, 3H.

### Exemple 3 : (+) Trans-3,4,4a,5,6,10b-hexahydro-9-carbamoyl-4-propyl-2H-naphto[1,2-b]-1,4-oxazine

2 g du produit titre de l'exemple 2 sont chromatographiés par chromatographie haute pression en phase normale sur support chiral (Chiracel AD®, éluant: éthanol 100%, débit: 5 ml/mn, détection 240 nm) pour donner 0,72 g de l'énantiomère le moins retenu. Après recristallisation du méthanol, sont obtenus 0,7g du produit titre (ee>99%).
P.F. : 200-202°C (MK)

### R.M.N.: ¹H (DMSO)d₆

7,94 ppm, s, 1H; 7,90 ppm, s, 1H (échangeable); 7,66 ppm, dd, 1H; 7,15 ppm, s, 1H (échangeable); 7,14 ppm, d, 1H; 4,20 ppm, d, 1H; 4,00 ppm, dd, 1H; 3,78 ppm, m, 1H; 2,98-2,70 ppm, m, 4H; 2,40-2,0 ppm, m, 4H; 1,60-1,35 ppm, m, 3H; 0,9 ppm, t, 3H.
[α]^{20°C}: (c= 1 %, CHCl₃).

| | | | | | |
|---|---|---|---|---|---|
| λ nm | 589 | 578 | 546 | 436 | 365 |
| α° | + 65,8 | + 68,5 | + 78,0 | + 131,2 | + 200,0 |

### Exemple 4 : (-)Trans-3,4,4a,5,6,10b-hexahydro-9-carbamoyl-4-propyl-2H-naphto[1,2-b]-1,4-oxazine

2 g du produit titre de l'exemple 2 sont chromatographiés par chromatographie haute pression en phase normale sur support chiral (Chiracel AD®, éluant: éthanol 100%, débit: 5 ml/mn, détection 240 nm) pour donner 0,71g de l'énantiomère le plus retenu. Après recristallisation du méthanol, sont obtenus 0,69 g du produit titre (ee>99%).
P.F. : 200-202°C (MK)

### R.M.N.: ¹H (DMSO)d₆

7,94 ppm, s, 1H; 7,90 ppm, s, 1H (échangeable); 7,66 ppm, dd, 1H; 7,15 ppm, s, 1H (échangeable); 7,14 ppm, d, 1H; 4,20 ppm, d, 1H; 4,00 ppm, dd, 1H; 3,78 ppm, m, 1H; 2,98-2,70 ppm, m, 4H; 2,40-2,0 ppm, m, 4H; 1,60-1,35 ppm, m, 3H; 0,9 ppm, t, 3H.
[α]^{20°C}: (c= 1 %, CHCl₃).

| | | | | | |
|---|---|---|---|---|---|
| λ nm | 589 | 578 | 546 | 436 | 365 |
| α° | - 65,4 | - 68,3 | - 77,6 | - 131,2 | -197,6 |

### Exemple 5 : Trans-3,4,4a,5,6,10b-hexahydro-9-(N-méthylcarbamoyl)-4-propyl-2H-naphto[1,2-b]-1,4-oxazine

### Stade A : chlorhydrate de l'acide trans-3,4,4a,5,6,10b-hexahydro-4-propyl-2H-naphto [1,2-b]-1,4-oxazine-9-carboxylique

68 ml d'acide chlorhydrique 36 % sont additionnés à température ambiante à une solution de 6,14 g du composé titre de l'exemple 1 dans 68 ml d'acide acétique glacial. Après 18 heures de reflux, le mélange réactionnel est tiré à sec. Le résidu solide est trituré dans l'éther éthylique, filtré et séché pour donner 8,65 g d'une poudre blanche qui est recristallisée de 80 ml d'eau. On obtient 6,9 g du produit désiré.
P.F. : 260° C (K)
Rendement : 89 %

### Stade B : composé titre de l'exemple

La monométhylamine est introduite en 1 heure, à température ambiante, dans une solution de 1 g du composé obtenu précédemment, 1,13 g de 2(-1H-benzotriazol-1-yl)-1,1,3,3-tétraméthyluronium tétrafluoroborate (TBTU), et de 1,1 ml de triéthylamine dans 14 ml de chlorure de méthylène. Après 18 heures d'agitation à température ambiante, le mélange réactionnel est dilué avec du chlorure de méthylène, lavé une fois par NaOH 1N, trois fois par H₂O, séché sur sulfate de magnésium puis concentré sous vide. Le résidu solide (1,14 g) est recristallisé dans 100 ml d'acétate d'éthyle pour donner 0,41 g du composé titre.
P.F. : 179-180° C
Rendement : 44 %

### R.M.N.: ¹H 300MHz (CDCl₃)

7,8 ppm, s, 1H; 7,7 ppm, d, 1H; 7,1 ppm, d, 1H; 6,2 ppm, m, 1H (échangeable); 4,3 ppm, d, 1H; 4,1-3,9 ppm, m, 2H; 2,98 ppm, s, 3H; 2,9 ppm, m, 2H; 2,9 ppm ,m, 1H; 2,8-2,45 ppm, m, 2H; 2,3 ppm, m, 2H; 2,3-1,6 ppm, m, 2H; 1,5 ppm, m, 2H; 0,9 ppm, t, 3H.

### Exemple 6 : Trans-3,4,4a,5,6,10b-hexahydro-9-(N,N-diméthylcarbamoyl)-4-propyl-2H-naphto [1,2-b]-1,4-oxazine

1 g du produit obtenu à l'exemple 5, stade A est traité comme décrit à l'exemple 5, stade B (en utilisant la diméthylamine à la place de la monométhylamine) pour donner après concrétisation dans l'éther isopropylique 0,46 g de produit titre.
P.F. : 85-87°C (MK)
Rendement : 47 %

### R.M.N.: ¹H 300MHz (CDCl₃)

7,6 ppm, s, 1H; 7,2 ppm, d, 1H; 7,1 ppm, d, 1H; 4,30 ppm, d, 1H; 4,1-3,9 ppm, m, 2H; 3,05 ppm, s, 6H; 2,9 ppm ,m, 2H; 2,9 ppm ,m, 1H; 2,8-2,5 ppm, m, 2H; 2,2 ppm, m, 2H; 2,3-1,5 ppm, m, 2H; 1,5 ppm, m, 2H; 0,9 ppm, t, 3H.

### Exemple 7 : Trans-3,4,4a,5,6,10b-hexahydro-9-(N-butylcarbamoyl)-4-propyl-2H-naphto [1,2-b]-1,4-oxazine

A température ambiante, 1 g du produit obtenu à l'exemple 5, stade A, 1,13 g de TBTU et 0,26 g de n-butylamine sont mis en suspension dans 14 ml de chlorure de méthylène. 1,1 ml de triéthylamine sont additionnés à température ambiante et le mélange réactionnel est agité 18 heures à cette température. Le mélange réactionnel est dilué avec du chlorure de méthylène, lavé une fois par NaOH 1N, trois fois par H₂O, séché sur sulfate de magnésium puis concentré sous vide. Le résidu huileux est concrétisé dans l'éther isopropylique pour donner 0,6 g du produit titre.
P.F. : 128-131° C (MK)
Rendement : 56 %

### R.M.N.: ¹H 300MHz (CDCl₃)

7,8 ppm, s, 1H; 7,7 ppm, d, 1H; 7,15 ppm, d, 1H; 6,15 ppm, t, 1H (échangeable); 4,1-3,95 ppm, m, 2H; 3,4 ppm, q, 2H; 3,0 ppm, m, 2H; 2,9 ppm ,m, 1H; 2,85-2,5 ppm, m, 2H; 2,3 ppm, m, 2H; 2,3-1,6 ppm, m, 2H; 1,6-1,4 ppm, m, 6H; 0,9 ppm, t, 3H.

### Exemple 8 : Trans-3,4,4a,5,6,10b-hexahydro-9-(N-p-méthoxyphénylcarbamoyl)-4-propyl-2H-naphto[1,2-b]-1,4-oxazine

A température ambiante, 0,8 g du produit obtenu à l'exemple 5, stade A, 0,9 g de TBTU, une quantité catalytique de 4-diméthylaminopyridine et 0,35 g de p-méthoxyaniline sont mis en suspension dans 11 ml de chlorure de méthylène. 0,9 ml de triéthylamine sont additionnés à température ambiante et le mélange réactionnel est agité 18 heures à cette température. Le mélange réactionnel est dilué avec du chlorure de méthylène, lavé une fois par NaOH 1N, trois fois par H₂O, séché sur sulfate de magnésium puis concentré sous vide. Le résidu solide est recristallisé de l'acétate d'éthyle pour donner 0,42 g du produit titre.
P.F. : 185-186° C (MK)
Rendement : 43 %

### R.M.N.: ¹H 300MHz (CDCl₃)

7,92 ppm, s, 1H; 7,85 ppm, d, 1H; 7,8 ppm, m, 1H (échangeable); 7,5 ppm, d, 2H; 7,2 ppm, d, 1H; 6,9 ppm, d, 2H; 4,3 ppm, d, 1H; 4,1-3,45 ppm, m, 2H; 3,8 ppm, s, 3H; 2,95 ppm ,m, 1H; 2,9 ppm, m, 2H; 2,8-2,5 ppm, m, 2H; 2,3 ppm, m, 2H; 2,3-1,6 ppm, m, 2H; 1,6 ppm, m, 2H; 0,9 ppm, t, 3H.

### Exemple 9 : Trans-3,4,4a,5,6,10b-hexahydro-9-(N-p-méthoxybenzyl-carbamoyl)-4-propyl-2H-naphto[1,2-b]-1,4-oxazine

0,7 g du produit obtenu à l'exemple 5, stade A est traité comme décrit à l'exemple 7, (en utilisant la p-méthoxybenzylamine à la place de la n-butylamine) pour donner après recristallisation de l'acétate d'éthyle 0,34 g de produit titre.
P.F. : 148-150° C (MK)
Rendement : 38 %

### R.M.N.: ¹H 300MHz (CDCl₃)

7,85 ppm, d, 1H; 7,75 ppm, dd, 1H; 7,3 ppm, d, 2H; 7,2 ppm, d, 1H; 6,9 ppm, d, 2H; 6,4 ppm, t, 1H (échangeable); 4,6 ppm, m, 2H; 4,3 ppm, d, 1H; 4,1 -3,95 ppm, m, 2H; 3,8 ppm, s, 3H; 2,9 ppm, m, 4H; 2,3 ppm, m, 4H; 1,5 ppm, m, 3H; 0,9 ppm, t, 3H.

### Exemple 10 : Trans-3,4,4a,5,6,10b-hexahydro-9-éthoxycarbonyl-4-propyl-2H-naphte[1,2 -b]-1,4-oxazine et son chlorhydrate

1 g du composé titre de l'exemple 1 est mis en solution dans un mélange de 15 ml de chloroforme et 15 ml d'éthanol. La solution est refroidie à 0°C et un courant d'acide chlorhydrique gazeux est introduit bulle à bulle pendant 1 heure. Le mélange est ramené à température ambiante et agité 24 heures. Le mélange est alors concentré sous vide, repris par 100 ml de tampon phosphate et agité 3 jours à température ambiante. La solution est basifiée par de la soude 1N et extraite au chlorure de méthylène. Après évaporation on obtient 1 g de produit titre qui est transformé en son chlorhydrate par une solution 4N d'éther chlorhydrique. Poids : 1.05 g.
P.F. : 220-221° C (MK)
Rendement : 79 %

### RMN : ¹H 300MHz (CDCl₃)

13,20 ppm, s.large, 1H (échangeable); 8,18 ppm, s, 1H; 7,92 ppm, dd, 1H; 7,18 ppm, d, 1H; 8,35 ppm, d, 1H; 4,70 ppm, d, 1H; 4,38 ppm, q, 2H; 4,22 ppm, dd, 1H; 3,55 ppm, d, 1H; 3,33 ppm, td, 1H; 3,00 ppm, m, 5H; 2,57 ppm, m, 1H; 2,40 ppm, m, 1H; 2,02 ppm, m, 1H; 1,80 ppm, m, 1H; 1,40 ppm, t, 3H; 1,05 ppm, t, 3H.

### Exemple 11 : Trans-3,4,4a,5,6,10b-hexahydro-9-acétyl-4-propyl-2H-naphto[1,2-b]-1,4-oxazine et son chlorhydrate

1,3 g du composé obtenu au stade A de l'exemple 1 est mis en solution dans 10 ml de DMF. On ajoute à température ambiante 0,57 ml de triéthylamine, 2,22 ml de n-butylvinyl éther, 42 mg de 1,3 bis (diphénylphosphino) propane et 19,3 mg de 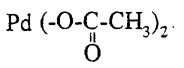 La solution est chauffée 3 heures à 80-90° C, puis après retour à température ambiante 15 ml d'HCl 1N sont ajoutés. L'agitation est maintenue 1 heure. Le mélange réactionnel est alors dilué à l'HCl 1N et la solution est lavée plusieurs fois à l'éther. La phase aqueuse est alcalinée à la soude et extraite quatre fois par du chlorure de méthylène. Après évaporation et purification par chromatographie flash (CH₂Cl₂/CH₃COOC₂H₅ : 90/10 et 80/20) on obtient 0,61g de produit attendu dont on prépare le chlorhydrate par action de l'éther chlorhydrique. Poids : 0,57 g.
P.F. : 225-226° C (M.K.)
Rendement : 54 %

### RMN : ¹H 300MHz (DMSO)

11,58 ppm, s, 1H (échangeable); 8,03 ppm, s, 1H; 7,88 ppm, d, 1H; 7,33 ppm, d, 1H; 5,01 ppm, d, 1H; 4,22 ppm, d, 2H; 3,58 ppm, d, 1H; 3,40 à 3,20 ppm, m, 4H; 3,15 à 2,90 ppm, m, 3H; 2,55 ppm, s, 3H; 2,04 ppm, m, 1H; 1,72 ppm, m, 2H; 0,98 ppm, t, 3H.

### Exemple 12 : Etude pharmacologique

### A. In vitro : étude de la liaison aux récepteurs humains D₂ et D₃

### ◆ Culture cellulaire

Les cellules CHO (Chinese Hamster Ovary) ont été transfectées de façon stable par le gène codant pour le récepteur humain de la dopamine D₂ ou D₃ selon les méthodes connues de la littérature. Les cellules natives sont déficientes en enzyme DHFR (DiHydroFolate Reductase). Ces cellules sont cultivées dans une étuve à 37° C en atmosphère humide 5 % CO₂, 95 % air. Les transfections ont été réalisées en utilisant la Lipofectine (Gibco). Les cellules CHO cotransfectées avec le récepteur D₂ humain et le gène de résistance à la phléomycine ont été sélectionnées pour leur résistance à la présence de cet antibiotique dans le milieu de culture. Les cellules transfectées avec le récepteur D₃ humain ont été sélectionnées dans un milieu dépourvu d'hypoxanthine/thymidine, en présence de méthotréxate. La composition des milieux de culture utilisés sont pour les CHO-D₂ : DMEM (Dulbecco's Modified Eagle Medium) supplémentés à 10 % en sérum de veau foetal et en hypoxanthine/thimidine et pour les CHO-D₃ : DMEM supplémentés à 10 % en sérum de veau foetal dialysé. Les cellules sont récoltées à confluence et les membranes sont alors préparées.

### ◆ Préparation membranaire

Après quelques minutes en présence de trypsine 0,2 %, les cellules sont récupérées et centrifugées à 2 000g pendant 5 minutes. Le culot cellulaire, resuspendu dans du tampon Tris-HCl 10 mM, pH 7,5 contenant 5mM de MgSO₄, est alors passé au Polytron®. L'homogénat est ensuite centrifugé à 50 000g pendant 15 minutes, et le culot est resuspendu par sonication douce dans le tampon d'incubation de composition : 50 mM de tris-HCl, pH 7,5 contenant 120 mM de NaCI, 5 mM de KCl, 2 mM de CaCl₂, 5 mM de MgCl₂. Les membranes sont ensuite aliquotées et conservées à -80° C jusqu'au jour de l'expérience.

### ◆ expériences de liaison

Les incubations sont effectuées dans des tubes en polypropylène pour un volume final de 400 µl contenant
100 µl de [¹²⁵I]-iodosulpride (Amersham)
   à 0,1 et 0,2 nM pour les récepteurs D₂ et D₃ respectivement.
100 µl de tampon (tubes totaux)
ou 100 µl de raclopride 10 µM (liaison non spécifique)
ou 100 µl de composé
200 µl de préparation membranaire contenant les récepteurs D₂ ou D₃, dans du tampon additionné de 0,2 % de BSA (Bovine Serum Albumine).

Les gammes de concentration de chaque composé comportent au moins sept points déterminés en triple. Chaque expérience est répétée au moins deux fois.

L'incubation qui dure trente minutes à 30° C est stoppée par une filtration rapide sur appareil Brandie, suivie de trois rinçages consécutifs avec du tampon Tris-HCl pH 7,4 contenant 120 mM de NaCl. Les filtres récupérés sont ensuite comptés au compteur gamma.

### ◆ Analyse des résultats

L'IC₅₀ représentant la concentration donnant 50 % d'inhibition de la liaison du radioligand est calculée par régression non linéaire (méthode Prism Graph).

La valeur de K₁ est dérivée de la formule K₁ = IC₅₀/(1+L/Kd) où L est la concentration de [¹²⁵I]-iodosulpride utilisée dans l'expérience et Kd sa constante de dissociation. Les résultats sont exprimés sous la forme de pK₁ (pK₁ = -logK₁).

Pour les récepteurs humains D₂ et D₃, les Kd sont respectivement égaux à 0,5 et 1,31 nM.

### ◆ Résultats

A titre d'exemple le produit de l'exemple 3 à un pK₁ de 8 pour le récepteur D₃ et de 6,5 pour le récepteur D₂.

### B. In vivo

### 1. Test d'enregistrement de l'activité électrique unitaire, extracellulaire, dans l'aire tegmentale ventrale du rat.

### ◆ Principe

L'administration d'un agoniste dopaminergique diminue d'une manière dose-dépendante la fréquence de décharge des neurones. Cet effet est réversé par l'halopéridol, antagoniste dopaminergique.

### ◆ Méthode

Les rats sont anesthésiés à l'hydrate de chloral (400 mg/kg, i.p.) et placés dans un appareil de stéréotaxie (Unimécanique, France) après cathétérisation de la veine fémorale. Le niveau d'anesthésie est maintenu par une administration i.p. de l'hydrate de chloral toutes les heures ; la température rectale est conservée à 37±1° C par une couverture chauffante thermostatée. Une microélectrode en tungstène (10 MΩ, 1µm) est descendue à l'aide d'un microdescendeur électronique (Unimécanique, France) au niveau de l'aire tegmentale ventrale (AP : -5.5 / bregma ; L : 0.7 ; H : 7.0-8.5 / dura ; atlas de Paxinos et Watson, 1986). Les potentiels de cellules dopaminergiques sont reconnus par leur morphologie (potentiels triphasiques +/-/+, d'une durée supérieure à 3 msec), leur rythme de décharge, soit régulier, soit en bouffées d'amplitude décroissante, et leur fréquence de décharge comprise entre 2 et 8 Hz. On enregistre une seule cellule par animal.

Après une période ≥ 5 min. (activité basale) et une première injection de véhicule (eau distillée additionnée de quelques gouttes d'acide lactique dilué ; pH ramené à 5 par NaOH 1N), les produits de l'invention sont administrés par voie intraveineuse en doses cumulées croissantes avec un intervalle de 2-3 min.

### ◆ Analyses des résultats

L'acquisition des données est faite par le logiciel Spike2 (Cambridge Electronic Design, England). La fréquence de décharge est mesurée sur une minute au maximum de variation entre chaque injection et exprimée en pourcentage de variation par rapport à l'activité basale (moyenne des 5 minutes précédant le premier traitement) définie comme 100 %. L'effet des produits est évalué statistiquement par une analyse de variance sur mesures répétées suivie d'un test de Newman-Keuls pour comparaison des effets des différentes doses à l'effet du véhicule.

### ◆ Résultats

A titre d'exemple le tableau suivant montre les effets du produit de l'exemple 3.

| **Doses** µg/kg i.v. | 0 | 0.125 | 0.25 | 0.5 | 1 | 2 | 4 | **halopéridol** 16 µg/kg i.v. |
|---|---|---|---|---|---|---|---|---|
| Ex. 3 | 96,7±0,24 | 82,8±1,26∗ | 76,8±1,76∗ | 59,0±3,76∗ | 33,6±5,25∗ | 14,4±4,38∗ | 0.00∗ | 102,3±14.0 |

| | | | | | | | | |
|---|---|---|---|---|---|---|---|---|
| *Valeurs individuelles (n = 4) = Moyenne. ± erreur standard à la moyenne. Comparaison des effets des différentes doses du composé par analyse de variance sur mesures répétées : Exemple 3, F(6,18) = 166,p < 0.001. Test de Newman-Keuls : ∗ = p ≤ 0,001.* | | | | | | | | |

### 2. Test de vocalisations ultrasoniques chez le rat

### ◆ Principe

Lorsqu'un rat est placé dans un environnement associé préalablement à une expérience désagréable (chocs électriques sur les pattes), son anxiété se traduit par l'émission de cris non audibles (ou vocalisations ultrasoniques). L'activité anxiolytique d'un produit se manifeste par une réduction de la durée de ces vocalisations.

### ◆ Appareillage

Des boîtes standard (Coulboum Instruments), placées dans des caissons insonorisants et ventilés, sont équipées d'un sol constitué de barreaux métalliques électrifiables (générateur de chocs et scrambler Med Associates Inc) et d'un microphone situé au centre du plafond. Les ultrasons sont transformés dans une gamme audible (bat-detector Buitenbedrijf). Les signaux ainsi modifiés sont filtrés puis traités (logiciel RTS, Engineering Design). Les spectrogrammes obtenus sont enregistrés sur bandes DAT.

### ◆ Méthode

Des rats mâles de souche Wistar pesant 180-200 g à leur arrivée sont logés par cage de quatre avec libre accès à l'eau et à la nourriture, de cinq jours avant le début de l'étude jusqu'à la fin de celle-ci. La procédure employée se décompose en trois étapes successives séparées par 24h, appelées entraînement, sélection et test. Durant la séance d'entraînement, les animaux sont placés individuellement dans les boîtes et y reçoivent six chocs électriques (0,8 mA, 8 s) distribués aléatoirement sur une période de sept minutes. La sélection consiste à placer chaque animal dans une boîte pendant deux minutes pour y recevoir un seul choc, et à l'y replacer trente minutes plus tard pour une séance d'enregistrement des vocalisations ultrasoniques de dix minutes ; les animaux dont la durée des vocalisations est inférieure à 90 secondes sont écartés de la suite de l'expérience. La phase de test se déroule de manière similaire à l'étape de sélection, les produits ou le véhicule étant en outre administrés à la fin de la séance de deux minutes.

### ◆ Résultats

A titre d'exemple, le tableau suivant montre les effets du produit de l'exemple 3 administré par voie s.c. sous un volume de 1 ml/kg

| **dose** mg/kg s.c. | **durée des vocalisations ultrasoniques (s)** moyenne ± e.s.m. (n) |
|---|---|
| | Exemple 3 |
| 0 | 251 ∓ 29 (16) |
| 0.0025 | 163 ± 54 (7) |
| 0.01 | 144 ± 79 (5) |
| 0.04 | 17 ± 13 (6) ∗∗ |
| 0.16 | 0,3 ± 0,3 (4) ∗∗ |

| | |
|---|---|
| *e.s.m. : erreur standard à la moyenne* *n : nombre de rats* *comparaison versus véhicule (test de Dunnett) :* ∗∗ *p < 0.01* | |

Aux doses de 0,04 et 0,16 mg/kg, ce produit entraîne une diminution importante de la durée des vocalisations, ce qui traduit son activité anxiolytique.

### 3. Test de la nage forcée

### ◆ Principe

Le test de la nage forcée (Porsolt R. *et al, Eur. J. Pharmacol*., **1978**, 47, 379-91) est un test comportemental qui consiste à induire chez le rat un état de "désespoir", en plaçant l'animal naïf pendant quinze minutes dans une enceinte remplie d'eau, d'où il ne peut s'échapper. Pendant les cinq à dix premières minutes, le rat se débat vigoureusement pour finalement adopter une posture immobile pendant la fin de l'essai. Placé le jour suivant dans la même enceinte, l'animal reste immobile pendant la majeure partie du test (durée min). Les antidépresseurs réduisent la durée d'immobilité du rat, lors du test.

### ◆ Expérimentation

L'expérience s'effectue en deux jours, à 24 heures d'intervalle, sur des rats d'un poids moyen de 170 g, stabulés la veille en cages individuelles, avec libre accès à la boisson et à la nourriture.

Le premier jour, chaque rat est placé pendant quinze minutes, dans un cylindre en verre (20 cm de diamètre x 40 cm de haut) rempli d'eau maintenue à 25° C, sur une hauteur de 15 cm. Le deuxième jour, l'animal est placé à nouveau dans un cylindre pendant cinq minutes ; le temps total (en sec) d'immobilité du rat est mesuré. Le produit ou le solvant est administré à l'animal trente minutes avant le début du test.

### ◆ Résultats

A titre d'exemple et pour illustrer l'activité des produits de l'invention, les effets du produit de l'exemple 3 sont rapportés dans le tableau suivant.

| **produit** | **dose** mg/kg s.c. | **immobilité (sec)** moyenne ± e.s.m. (n) |
|---|---|---|
| Témoins (eau distillée) | 0 | 172,2 ± 19,87 (8) |
| } | 0.04 | 183,14 ± 4,06 (5) |
| } | 0.16 | 170,71 ± 23,09 (5) |
| Exemple 3 } | 0.31 | 130,33 ± 18,67 (5) |
| } | 0.63 | 36,87±22,28^{∗} (5) |
| } | 10.0 | 0,27 ± 0,27^{∗} (5) |
| *(n) = nombre de rats* | | |

Le produit de l'exemple 3 réduit de façon dose-dépendante la durée d'immobilité de l'animal et présente donc un excellent effet antidépresseur avec une DI₅₀ = 0,41 mg/kg s.c.

### 4. Rotations induites par les agonistes dopaminergiques chez des rats avec lésion unilatérale de la Substantia nigra

### ◆ Principe

L'injection unilatérale de la neurotoxine 6-hydroxy-dopamine (6-OH-DA) dans la *Substantia nigra* produit une dégénérescence des voies ascendantes nigrostriées, avec hypersensibilité des récepteurs dopaminergiques post-synaptiques du côté lésé. Chez un rat ayant subi une telle lésion, l'administration systémique de produits agonistes directs (apomorphine) induit des rotations contralatérales (du côté opposé à la lésion). Ce test permet de mettre en évidence les propriétés dopaminergiques agonistes de produits à visée thérapeutique dans la maladie de Parkinson.

### ◆ Méthodes

**Lésion** : la lésion est effectuée sur des rats Wistar mâles de 280 à 330 g anesthésiés au pentobarbital (40-50 mg/kg i.p.) et ayant reçu une dose de 25 mg/kg i.p. de désipramine. L'animal est placé dans un appareil stéréotaxique KOPF, le crâne orienté selon l'Atlas de Pellegrino et Cushman (1979). Un volume de 4 µl d'une solution de 6-OH-DA (2 µg/µl) est injecté lentement au moyen d'un microperfuseur, au niveau de la *Substantia nigra* gauche, (A = 2,4 mm ; L = 2.0 mm ; V = 3.1 mm, par rapport au zéro interaural) (U. Ungerstedt, *Acta Physiol. Scandi. Suppl*., 1971, 367, 69-93)

**Matériel :** l'enregistrement du nombre et du sens des rotations se fait automatiquement sur ordinateur, grâce au système ROTACOUNT (Columbus Co, USA). L'animal est placé dans un cylindre à fond plat, de 30 cm de diamètre et 50 cm de hauteur. Un câble fin, semi-rigide, ceinture l'animal sous les pattes avant et se connecte à la cellule de comptage optique située au dessus du cylindre et reliée à l'ordinateur.

**Sélection des animaux lésés :** un mois après l'induction de la lésion par la 6-OH-DA, les animaux correctement lésés sont sélectionnés selon un critère d'au moins 150 rotations contralatérales effectuées en 1 heure après administration de l'agoniste dopaminergique, apomorphine (0,04 mg/kg, s.c.).

**Expérimentation :** les animaux sont testés une fois par semaine, avec administration des produits de l'invention en alternance avec celle de l'agoniste dopaminergique. L'enregistrement des rotations contralatérales débute dès l'injection de l'agoniste dopaminergique (T0) et dure 1 heure. Chaque animal est son propre contrôle.

### ◆ Résultats

A titre d'exemple, le tableau suivant montre les effets du produit de l'exemple 3 administré par voie s.c.

| **produit** | **dose** mg/kg s.c. | **rotations contralatérales** moyenne ± e.s.m. (n) |
|---|---|---|
| Contrôle Sérum | 0 | 11,0 ± 4,3 (23) |
| Contrôle Apomorphine | 0.04 | 531,0 ± 54,1 (14) |
| } | 0.01 | 84,5 ± 79,7 (2) |
| Exemple 3 } | 0.04 | 223,0 ± 103,5 (6) |
| } | 0.16 | 336,6 ± 85,5 (7) |
| *(n) = nombre de rats* | | |

Le produit de l'exemple 3 est actif dans ce test dès la dose de 0,04 mg/kg.

## Revendications

1. Les composés de formule I : 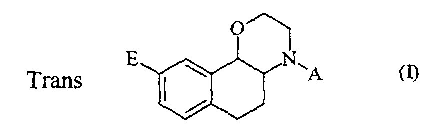 dans laquelle :
◆ A représente :
un radical (C₁-C₁₀)alkyle, (C₃-C₁₀)alcényle ou (C₃-C₁₀)alcynyle, chacun en chaîne droite ou ramifiée et chacun éventuellement substitué par un ou plusieurs radicaux cycloalkyles ayant de 3 à 8 atomes de carbone, ou par un radical aryle choisi parmi les radicaux phényle, thiényle et pyridyle chacun d'eux éventuellement substitué par un ou plusieurs substituants choisis parmi les atomes d'halogène, le radical hydroxy et les radicaux alkyle et alkoxy ayant chacun de 1 6 atomes de carbone en chaîne droite ou ramifiée.
◆ E représente : 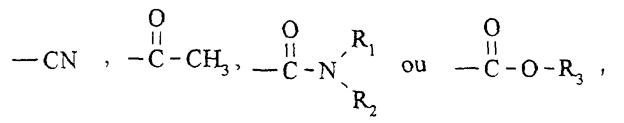 dans lesquels :
- R₁ et R₂, identiques ou différents, représentent chacun un atome d'hydrogène ou prennent la signification de A précédemment définie, et
- R₃ représente un atome d'hydrogène ou un radical alkyle de 1 à 5 atomes de carbone en chaine linéaire ou ramifiée ;
sous forme racémique ou d'isomères optiques et leurs sels d'addition avec un acide pharmaceutiquement acceptable.

2. Un composé de la revendication 1 qui est la trans-3,4,4a,5,6,10b-hexahydro-9-cyano-4-propyl-2H-naphto[1,2-b]-1,4-oxazine et son chlorhydrate

3. Un composé de la revendication 1 qui est la trans-3,4,4a,5,6,10b-hexahydro-9-carbamoyl-4-propyl-2H-naphto[1,2-b]-1,4-oxazine

4. Un composé de la revendication 1 qui est la (+) trans-3,4,4a,5,6,10b-hexahydro-9-carbamoyl-4-propyl-2H-naphto[1,2-b]-1,4-oxazine.

5. Le procédé de préparation des composés de la revendication 1 **caractérisé en ce que** :
- l'on fait réagir un composé trans de formule II : 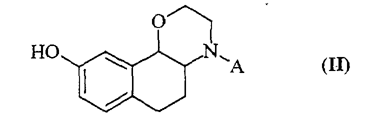 dans laquelle A a la signification définie dans la revendication 1,
- avec de l'anhydride triflique en présence de pyridine pour obtenir un composé trans de formule III : 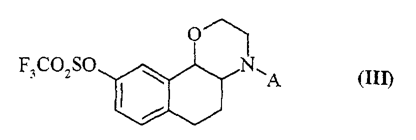 dans laquelle A a la signification précédemment définie,
- et l'on fait réagir ce composé :
. soit avec du cyanure de zinc et du tétrakis (triphénylphosphine)palladium (o) dans le diméthylformamide à chaud, pour obtenir un composé trans de formule la : 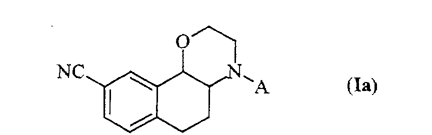 dans laquelle A a la signification précédemment définie,
. soit avec du n-butyl vinyl éther dans les conditions de la réaction de Heck pour obtenir un composé trans de formule Ib : 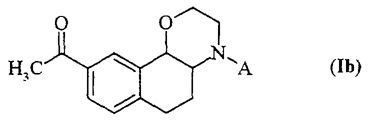
dans laquelle A a la signification précédemment définie,
et, si on le désire,
- le composé Ia, précédemment obtenu, est traité par un mélange d'acide chlorhydrique et d'acide acétique au reflux, pour obtenir un composé trans de formule Ic : 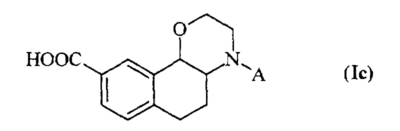 dans laquelle A a la signification précédemment définie,
- lequel composé Ic peut être traité :
. soit, par un composé de formule IV :
R₃―OH (**IV**)
dans laquelle R₃ a la signification définie dans la revendication 1,
en présence d'acide chlorhydrique, pour obtenir un composé trans de formule Id : 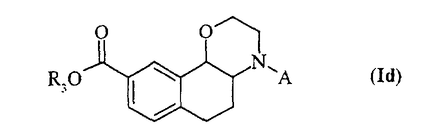 dans laquelle A et R₃ ont les significations précédement définies,
. soit par un composé de formule V : 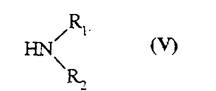 dans laquelle R₁ et R₂ ont les significations définies dans la revendication 1,
en présence de 2-(1H-benzotriazol-1-yl) 1,1,3,3-tétraméthyluronium tétrafluoroborate, de triéthylamine et d'une quantité catalytique de 4-diméthylaminopyridine dans le chlorure de méthylène, pour obtenir un composé trans de formule le : 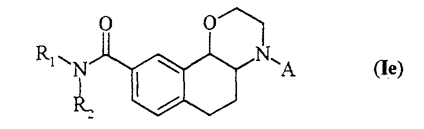 dans laquelle A, R₁, et R₂, ont les significations précédemment définies.

6. Les compositions pharmaceutiques, utilisables dans le traitement de maladies du système nerveux central, contenant comme principe actif un composé selon une quelconque des revendications 1 à 4, avec un ou plusieurs excipients pharmaceutiques appropriés.

## Patentansprüche

1. Verbindungen der Formel I: 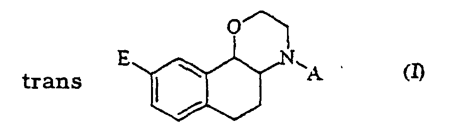 in der:
◆ A:
eine (C₁-C₁₀)-Alkylgruppe, (C₃-C₁₀)-Alkenylgruppe oder (C₃-C₁₀)-Alklnylgruppe darstellt, wobei jede dieser Gruppen geradkettig oder verzweigt und gegebenenfalls substituiert sein kann durch eine oder mehrere Cycloalkylgruppen mit 3 bis 8 Kohlenstoffatomen oder durch eine Arylgruppe ausgewählt aus Phenyl-, Thienyl- und Pyridylgruppen, welche gegebenenfalls substituiert sein können durch einen oder mehrere Substituenten ausgewählt aus Halogenatomen, Hydroxygruppen und Alkyl- und Alkoxygruppen mit jeweils 1 bis 6 Kohlenstoffatomen in gerader oder verzweigter Kette bedeutet;
◆ E: 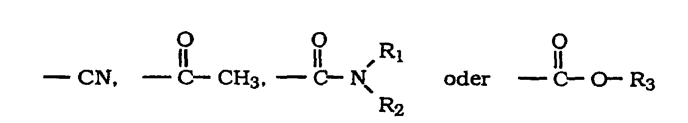 bedeutet, in denen:
- R₁ und R₂, die gleichartig oder verschieden sind, jeweils ein Wasserstoffatom bedeuten oder die oben definierten Bedeutungen von A besitzen, und
- R₃ ein Wasserstoffatom oder eine Alkylgruppe mit 1 bis 5 Kohlenstoffatomen in gerader oder verzweigter Kette darstellt;
in racemischer Form oder in Form der optischen Isomeren und deren Additionssalze mit einer pharmazeutisch annehmbaren Säure.

2. Verbindung nach Anspruch 1, nämlich trans-3,4,4a,5,6,10b-Hexahydro-9-cyano-4-propyl-2H-naphtho[1,2-b]-1,4-oxazin und dessen Hydrochlorid.

3. Verbindung nach Anspruch 1, nämlich trans-3,4,4a,5,6,10b-Hexahydro-9-carbamoyl-4-propyl-2H-naphtho[1,2-b]-1,4-oxazin.

4. Verbindung nach Anspruch 1, nämlich (+)-trans-3,4,4a,5,6,10b-Hexahydro-9-carbamoyl4-propyl-2H-naphtho[1,2-b]-1,4-oxazin.

5. Verfahren zur Herstellung der Verbindungen nach Anspruch 1, **dadurch gekennzeichnet, daß** man:
- eine trans-Verbindung der Formel II: 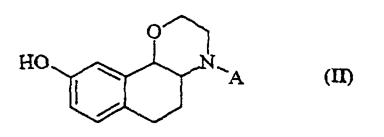 in der A die in Anspruch 1 angegebenen Bedeutungen besitzt,
- mit Trifluormethansulfonsäureanhydrid in Gegenwart von Pyridin umsetzt zur Bildung einer trans-Verbindung der Formel III: 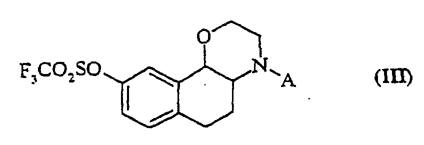 in der A die oben angegebenen Bedeutungen besitzt,
- und man diese Verbindung:
. entweder mit Zinkcyanid und Tetrakis(triphenylphosphin)-palladium-(0) in warmem Dimethylformamid umsetzt zur Bildung einer trans-Verbindung der Formel Ia: 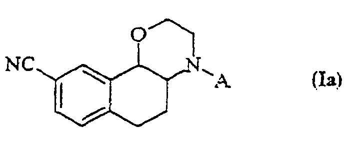 in der A die oben angegebenen Bedeutungen besitzt,
. oder mit n-Butylvinylether unter den Bedingungen der Heck-Reaktion umsetzt zur Bildung einer trans-Verbindung der Formel Ib: 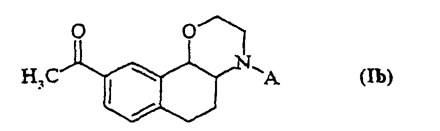 in der A die oben angegebenen Bedeutungen besitzt,
und gewünschtenfalls
- die in der obigen Weise erhaltene Verbindung Ia mit einer Mischung aus Chlorwasserstoffsäure und Essigsäure am Rückfluß behandelt zur Bildung einer trans-Verbindung der Formel Ic: 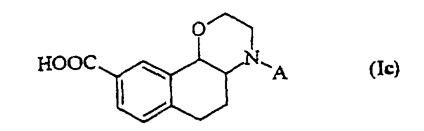 in der A die oben angegebenen Bedeutungen besitzt,
- welche Verbindung Ic man:
. entweder mit einer Verbindung der Formel IV:
R₃―OH (IV)
in der R₃ die in Anspruch 1 angegebenen Bedeutungen besitzt,
in Gegenwart von Chlorwasserstoffsäure zur Bildung einer trans-Verbindung der Formel Id: 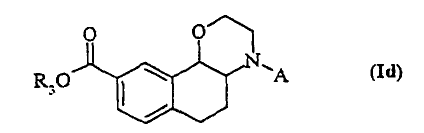 in der A und R₃ die oben angegebenen Bedeutungen besitzen,
. oder mit einer Verbindung der Formel V: 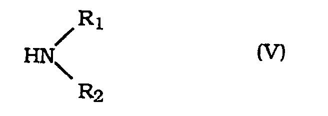 in der R₁ und R₂ die in Anspruch 1 angegebenen Bedeutungen besitzen,
in Gegenwart von 2-(1H-Benzotriazol-1-yl)-1,1,3,3-tetramethyluroniumtetrafluorborat, Triethylamin und einer katalytischen Menge 4-Dimethylaminopyridin in Methylenchlorid umsetzt zur Bildung einer trans-Verbindung der Formel Ie: 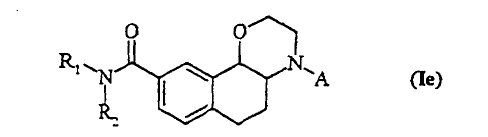 in der A, R₁ und R₂ die oben angegebenen Bedeutungen besitzen.

6. Pharmazeutische Zubereitungen für die Behandlung von Erkrankungen des Zentralnervensystems enthaltend als Wirkstoff eine Verbindung nach einem der Ansprüche 1 bis 4 zusammen mit einem oder mehreren pharmazeutisch geeigneten Trägermaterialien.

## Claims

1. The compounds of formula I : 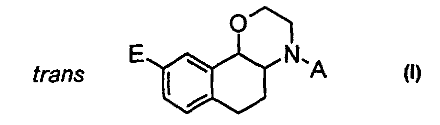 wherein:
◆ A represents:
a (C₁-C₁₀)alkyl, (C₃-C₁₀)alkenyl or (C₃-C₁₀)alkynyl radical each of which is straight-chain or branched and optionally substituted by one or more cycloalkyl radicals having from 3 to 8 carbon atoms, or by an aryl radical selected from the radicals phenyl, thienyl and pyridyl each of which is optionally substituted by one or more substituents selected from the halogen atoms, hydroxy radicals, and alkyl and alkoxy radicals each of which has from 1 to 6 carbon atoms and is straight-chain or branched,
◆ E represents: 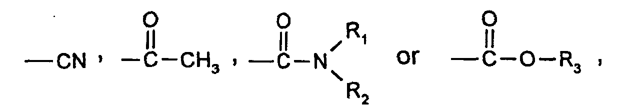 wherein:
- R₁ and R₂, which may be identical or different, each represents a hydrogen atom or has the meaning given for A above, and
- R₃ represents a hydrogen atom or a straight-chain or branched alkyl radical having from 1 to 5 carbon atoms;
in racemic form or in the form of optical isomers, and the addition salts thereof with a pharmaceutically acceptable acid.

2. A compound of claim 1 which is *trans*-3,4,4a,5,6,10b-hexahydro-9-cyano-4-propyl-2H-naphtho[1,2-b]-1,4-oxazine and its hydrochloride.

3. A compound of claim 1 which is *trans*-3,4,4a,5,6,10b-hexahydro-9-carbamoyl-4-propyl-2H-naphtho[1,2-b]-1,4-oxazine.

4. A compound of claim 1 which is (+)-*trans*-3,4,4a,5,6,10b-hexahydro-9-carbamoyl-4-propyl-2H-naphtho[1,2-b]-1,4-oxazine.

5. A process for the preparation of compounds of claim 1, **characterised in that**:
- a *trans* compound of formula II : 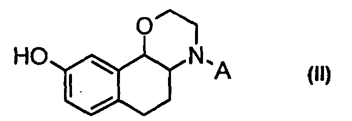 wherein A is as defined in claim 1,
- is reacted with triflic anhydride in the presence of pyridine to obtain a *trans* compound of formula III : 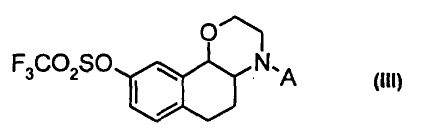 wherein A is as defined hereinbefore,
- and that compound is reacted :
. either with zinc cyanide and tetrakis(triphenylphosphine)palladium(0) in dimethylformamide at elevated temperature to obtain a *trans* compound of formula Ia : 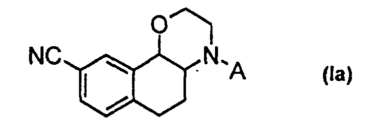 wherein A is as defined hereinbefore,
. or with n-butyl vinyl ether under Heck reaction conditions to obtain a *trans* compound of formula lb : 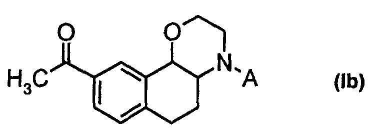 wherein A is as defined hereinbefore,
and, if desired,
- the compound Ia, obtained previously, is treated with a mixture of hydrochloric acid and acetic acid at reflux to obtain a *trans* compound of formula Ic : 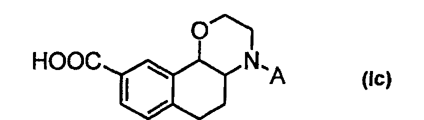
wherein A is as defined hereinbefore,
- which compound Ic may be treated :
. either with a compound of formula IV :
R₃-OH (IV)
wherein R₃ is as defined in claim 1,
in the presence of hydrochloric acid, to obtain a *trans* compound of formula Id : 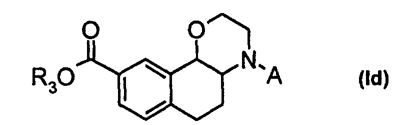 wherein A and R₃ are as defined hereinbefore,
. or with a compound of formula V: 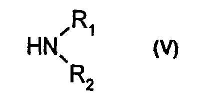
wherein R₁ and R₂ are as defined in claim 1,
in the presence of 2-(1H-benzotriazol-1-yl)-1,1,3,3-tetramethyluronium tetrafluoroborate, triethylamine and a catalytic amount of 4-dimethylaminopyridine in methylene chloride, to obtain a *trans* compound of formula le : 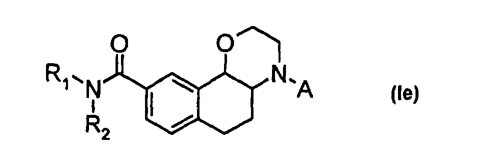 wherein A, R₁ and R₂ are as defined hereinbefore.

6. Pharmaceutical compositions, which can be used in the treatment of disorders of the central nervous system, comprising as active ingredient a compound according to any one of claims 1 to 4 together with one or more appropriate pharmaceutical excipients.
